# EUROPEAN PATENT SPECIFICATION

(11) **EP 2 550 372 B1**
(45) Date of publication and mention of the grant of the patent: **08.01.2014**
(21) Application number: 10726890.6
(22) Date of filing: 28.04.2010
(51) Int. Cl.: C12Q 1/70

(54) **KIT FOR DETECTION OF HUMAN PAPILLOMAVIRUS**
KIT ZUR DETEKTION VON HPV
KIT POUR DETECTER HPV

(30) Priority: 24.03.2010 ES 201000392
(43) Date of publication of application: 30.01.2013
(73) Proprietor: Genomica S.A.U., 28823 Coslada, Madrid (ES)
(72) Inventor: VILLAHERMOSA JAEN, Maria, Luisa, E-28823 Coslada, Madrid (ES); MORAGA QUINTANILLA, Ana, Isabel, E-28823 Coslada, Madrid (ES)
(74) Representative: Fleck, Barbara
(86) International application number: PCT/EP2010/002598
(87) International publication number: WO 2011/116797

(56) References cited:
- WO-A2-2006/077102
- WO-A2-2007/017699
- DATABASE Geneseq [Online] 29 May 2008 (2008-05-29), "HPV-33 detection probe, SEQ ID 4.", XP002610430, retrieved from EBI accession no. GSN:AJF59106 Database accession no. AJF59106 & WO 2007/100198 A1 (AHN WOONG SHICK [KR]; HAN BYOUNG-DON [KR]; OH YONG TAEK [KR]; CHUN SUN) 7 September 2007 (2007-09-07)
- DATABASE Geneseq [Online] 5 May 2005 (2005-05-05), "Amidated HPV31 probe SEQ ID NO 8.", XP002610431, retrieved from EBI accession no. GSN:ADY29865 Database accession no. ADY29865

## Description

### Background of the invention.

Human papillomavirus (HPV) is one of the aetiological agents of cervix (neck of the uterus) cancer, being the causative agent of the most frequent sexual disease in women (Bosch et al., 1995, J. Natl. Cancer Inst., 87: 796-802). In recent years, HPV infection has been determined as being the main cause of cervix cancer and of intraepithelial cervix neoplasia (Walboomers et al., 1999, J. Path., 189:12-19; Bosch et al., 2002, J. Clin. Pathl., 55: 244-265).

Around 30 HPV types are sexually transmitted, and produce anogenital infection, these types being classified into two risk groups according to their association with cervix cancer (Dunne et al., 2007, JAMA, 297(8); Muñoz et al., 2003, N. Engl. J. Med., 348(6): 518-527): High and low oncogenic groups, respectively. An accurate identification of the HPV types that cause an infection is a main issue when the most adequate medical treatment is to be determined.

Of all HPV types, those which present the highest malignicity rate are types 16, 18, 31 and 33 (Berkhof et al., 2006, Cancer Epidemiol Biomarkers Prev., 15(7):1268-73). Further, all epidemiologic evidence points to the existence of a direct relationship between these HPV types and cervix cancer development (IARC Monographs on the evaluation of carcinogenics risk to humans. Human Papillomaviruses. Vol. 90. Lyon: International Agency for Research on Cancer, 2007).

Other studies indicate that HPV types 31 and 33 are, together with types 16 and 18, some of the most abundant types in cases of HSIL (high-grade squamous intraepithelial lesions) and SCC (squamous cell carcinoma of the cervix) (Clifford et al., 2003, British Journal of Cancer 89, 101-105).

The most frequently used and most sensitive molecular techniques for HPV detection are based on the Polymerase Chain Reaction or PCR; The two most commonly used PCR consensus reactions are the ones named MY-PCR, and its variant PG-MY, which contain the set of amplification primers MY11, MY09 and HMB01 (Manos et al., 1989, Cancer Cells, 7:209-214.; Hildesheim et al., 1994, J. Infect. Dis., 169: 235-240; Gravitt et al., 2000, J. Clin. Microbiol., 38(1): 357-361), as well as the one named GP-PCR, which contains the set of primers GP5+/GP6+ (de Roda et al., 1995, J. Gen. Virol., 76:1057-1062; Jacobs et al., 1995, J. Clin. Microbiol., 33: 901-905; Jacobs et al., 1997, J. Clin. Microbiol., 35:791-795).

Once the presence of HPV has been detected in a sample by means of an amplification reaction, the need to type it arises. Several techniques have been used with this purpose, such as DNA sequencing, restriction enzyme analysis and finally, techniques that are based on hybridisation of the PCR products with complementary probes immobilised on different surfaces (Jacobs et al., 1995, J. Clin. Microbiol., 33: 901-905).

A means of HPV diagnosis and typing is described in WO 2007/017699. According to this method, a PCR amplification reaction based on the primers MY-PCR takes place, followed by typing through hybridisation of the PCR product with a microarray of type-specific probes for a great variety of HPV types, both of high and low oncogenic risk.

Methods described in the art rely on using specific probes for the detection of specific HPV types. Therefore, it must be ensured that the probes used for detection hybridise specifically to the target HPV type and cross hybridisation to non-specific types must be avoided.

However, as shown herein, when using probes known in the art, cross hybridisation occurs, this behaviour not being based on sequence reasons. Specifically, probes intended for the specific detection of the HPV genotype 33 showed cross hybridisation to type 31 HPV.

The invention described herein is aimed at mitigating the shortcomings in the prior art.

### Summary of the invention.

The invention relates to the provision of a nucleic acid probe comprising or consisting of the sequence CTGTCACTAGTTACTTGTGTGCA (5'→3') (SEQ ID N° 3). As explained further herein, probes for the detection of type 33 HPV known in the art show hybridisation to type 31 HPV, thus leading to false positives. The inventors have surprisingly found that the nucleic acid sequence as defined in SEQ ID N° 3 is the only probe of a set of probes of similar characteristics that specifically hybridises to type 33 HPV and does not cross hybridise to type 31 HPV or to other genoptypes with a similar sequence. Thus, the probe as defined in SEQ ID N° 3 provides a solution to the problem of non-specific hybridisation of a probe intended for the detection of type 33 HPV to type 31 HPV.

With the nucleic acid probe as defined in SEQ ID NO 3, see also Table 1), non-specific hybridisation to genotype 31 HPV is avoided, as well as non-specific hybridisation to other HPV genotypes of a similar sequence. At the same time, specific hybridisation to type 33 HPV is attained.

Other newly designed probes that were designed following the same criteria as those for designing the probe of SEQ ID N° 3 (in particular, probes of SEQ ID N° 4 to 7 of Table 1), also did not show non-specific hybridisation to type 31 HPV, as shown by the control probes of SEQ ID N° 1 and 2. However, surprisingly, the ability to detect type 33 HPV itself was lost in these probes.

**Table 1.**

| **SEQ ID N°** | **Probe Name** | **Sequence (5'→3')** | **nt N°** | **Mₜ** | **%GC** |
|---|---|---|---|---|---|
| **3** | T33A2.1-A-AR | CTGTCACTAGTTACTTGTGTGCA | 23 | 66 | 43,5 |
| **4** | T33B1.2-A-AR | GTATATTTACCTAAGGGGTC | 20 | 56 | 40 |
| **5** | T33B1.3-A-AR | CCTTTTCCTTTGGAGGTACTG | 21 | 62 | 47,6 |
| **6** | T33B1.4-A-AR | GTATATTTACCTAAGGGGTCTTCC | 24 | 68 | 41,7 |
| **7** | T33B1.5-A-AR | CTTCCTTTTCCTTTGGAGGTACTG | 24 | 70 | 45,8 |

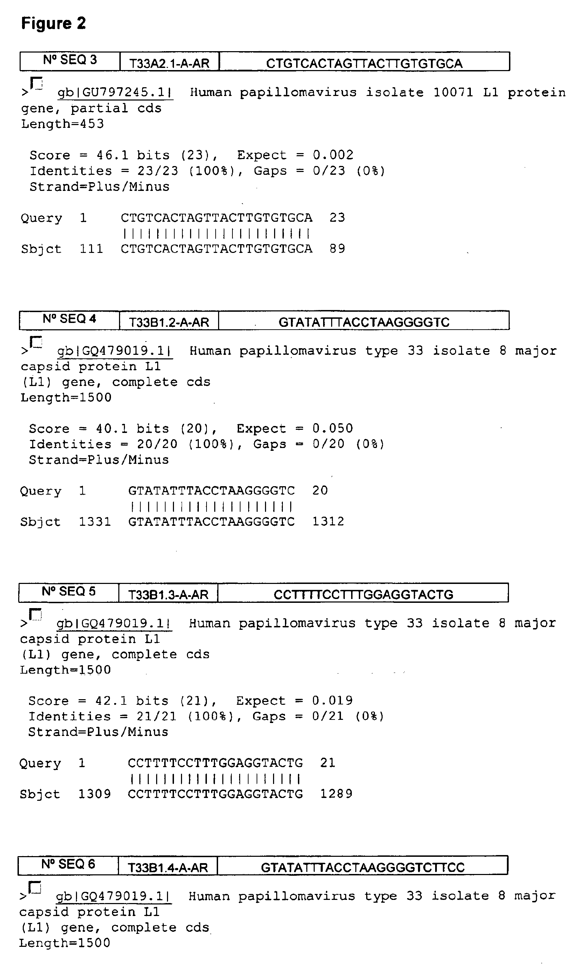

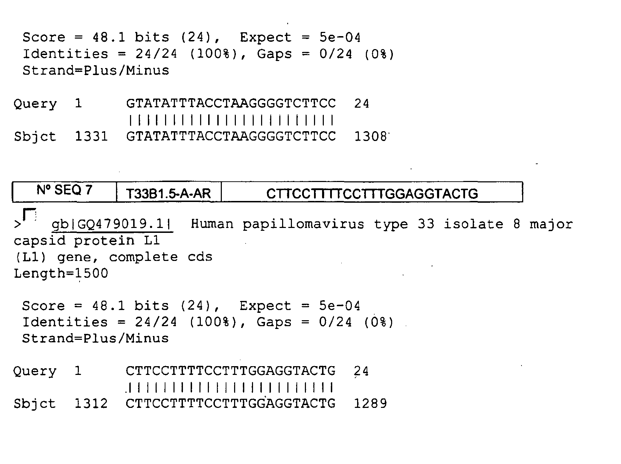

The reasons for the failure of SEQ ID N° 4 to 7 to specifically detect type 33 HPV are not known. However, the observation that genotype 33 of HPV cannot be detected with these is not due to the sequences of the probes. The inventors have shown in *in silico* analysis that these probes are perfectly complementary to the DNA sequence of type 33 HPV (Figure 2).

Further, the probe of SEQ ID N° 3 of the present invention, as well as the probes of SEQ ID N° 4 to 7, all share some very similar characteristics, in particular regarding probe length (Nucleotide number, nt N°), as well as Melting Temperature (Mₜ), GC percentage and other thermodynamic parameters.

Therefore, it was not expected, neither in view of the sequence alignment of Figure 2, nor in view of the characteristics of the newly designed probes (1), that the only probe that would prevent the non-specific hybridisation to type 31 HPV, as well as non-specific hybridisation to other HPV types of a similar sequence, but would specifically detect type 33 HPV, would be the probe of SEQ ID N° 3 of the present invention (CTGTCACTAGTTACTTGTGTGCA). Therefore, surprisingly, the inventors have found that the probe of SEQ ID N°. 3 can be used to specifically detect HPV 33 without cross hybridising to other HPV types.

Furthermore, although the probe of SEQ ID N° 3 is very similar to the control probe of SEQ ID N° 2 and merely lacks 2nt at the 5' end and 5nt at the 3' end (see Tables 1 and 2), it nevertheless does not exhibit the cross-hybridisation to type 31 HPV as shown by the control probe.

The inventors of the present invention have ascertained that slight variations of the sequence length of the probe of SEQ ID N° 3, in particular, variations of 1, 2, 3 or more nucleotides, affect the functional characteristics of the probe of SEQ ID NO 3 in a very relevant way.

In a first aspect, the present invention thus corresponds to a nucleic acid probe comprising or consisting of the sequence CTGTCACTAGTTACTTGTGTGCA (5'→3') (SEQ ID No. 3).

Another aspect of the present invention is the use of the nucleic acid probe comprising or consisting of sequence CTGTCACTAGTTACTTGTGTGCA (5'→3') (SEQ ID No. 3) for carrying out the specific detection of type 33 HPV.

The nucleic acid probe of the present invention may be comprised within a microarray of DNA probes, wherein probes for the specific detection of one or more HPV types may be present, and whose final purpose may be the detection and typing of HPV present in a sample.

Another aspect of the invention relates to a reliable method for the specific detection and/or identification of type 33 HPV in a clinical sample comprising using a nucleic acid probe comprising or consisting of sequence CTGTCACTAGTTACTTGTGTGCA (5'→3') (SEQ ID N° 3).

Yet another aspect of the present invention is a kit for the detection of one or more HPV types, the kit comprising a microarray comprising the nucleic acid probe comprising or containing the sequence CTGTCACTAGTTACTTGTGTGCA (5'→3') (SEQ ID N° 3).

Another aspect of the invention relates to an assay for detecting and typing HPV, the assay comprising performing a nucleic acid amplification reaction on a sample to amplify one or more HPV target sequence(s), obtaining single-standed oligonucleotides, allowing single stranded oligonucleotides to hybridise with one or more type specific HPV probes, wherein the one or more type specific HPV probes comprise a nucleic acid probe comprising or consisting of sequence CTGTCACTAGTTACTTGTGTGCA (5'→3') (SEQ ID N° 3). In one embodiment, the assay is for the detection of type 33 HPV, and comprises a nucleic acid probe comprising or consisting of sequence CTGTCACTAGTTACTTGTGTGCA (5'→3') (SEQ ID N° 3) only. In another embodiment, other probes as known in the art may be used in combination with SEQ ID N° 3.

### Brief description of the drawings.

Figure 1

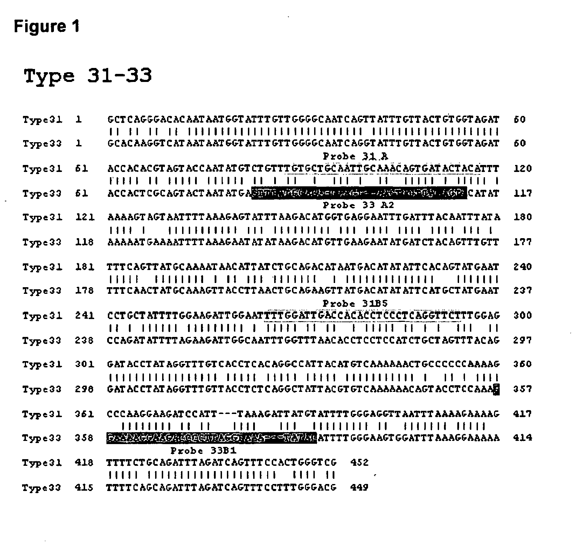

   Figure 1 shows the sequence alignment between the DNA sequences of HPV types 31 and 33.
   Within this figure, the position corresponding to the sequences with which the probes 33A2-AS and 33B1 (designed for the specific detection of HPV type 33) hybridise, is indicated.
   The position corresponding to the sequences with which the probes 31 B5 and 31A-AS (designed for the specific detection of HPV type 31) hybridise, is also indicated.
Figure 2
   Some representative examples of the *in silico* analysis of the probes of SEQ ID N° 3 to 7, and the sequences of the databases of GenBank are displayed.
Figure 3
   Visualisation of the hybridisation between the amplification product corresponding to a sample positive for type 31 and negative for type 33, and
   (i) a microarray that contained the original probes 33B1-AS and 33A2 for detection of type 33 (panel A), or
   (ii) a microarray that contained the probe according to the present invention, CTGTCACTAGTTACTTGTGTGCA (5'→3'), for detection of type 33 (panel B).

The probes for detection of type 31, that are contained within both microarrays, are probes 31A-AS and 31 B5.

Both the microarray of panel A, and the microarray of panel B, comprise position markers, that are surrounded by squares, as well as probes for the detection of DNA amplification control, named as "ADN".

Signals corresponding to the hybridisation signals of types 31 and 33 HPV are also indicated in this figure.

### Detailed description of the invention.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As shown in example 1, probes directed against type 33 HPV and which are known in the art also cross-hybriside to type 31 HPV. In order to solve the problem of the non-specific hybridisation of a probe intended to detect type 33 HPV to genotype 31 HPV, new probes were designed for detection of type 33 HPV. The program Oligo 6 (Molecular Biology Insights, Inc) was used with this purpose. Probes were of a shorter length than the probes known in the art (see for example 1, table 2), had a Guanidine/Citosine (G/C) content of 40-60%, anda Melting Temperature (Mt) with a maximum value of 15° C above the Hybridisation Temperature.

The resulting DNA probes are displayed on Table 1. SEQ ID N° 3 was shown to specifically hybridise to type 33 HPV and did not show cross hybridisation to other HPV types, including 31 HPV. Accordingly, the invention relates to a nucleic acid probe comprising or consisting of sequence CTGTCACTAGTTACTTGTGTGCA (5'→3') (SEQ ID N° 3). As shown herein, this probe may be used in the methods and kits of the invention alone or in combination with other probes which are specific probes for the detection of other HPV types. Such probes are known in the art, for example from WO 2007/017699.

Preferably, probes specific for at least 5, 10, 15, 20, 25, 30, 35, 40, or 42 HPV types are used in the methods and kits of the invention, which are preferably selected from HPV types 6, 11, 16, 18, 26, 30, 31, 32, 33, 34/64, 35, 39, 40, 42, 43, 44, 45, 51, 52, 53, 54, 56, 57, 58, 59, 61, 62, 66, 67, 68, 69, 70, 71, 72, 73, 74, 81, 82, 83, 84, 85 and 89. Preferably up to a total of 35 different HPV types are detected according to the different embodiments of the invention.

The nucleic acid sequence of SEQ ID N° 3 may further comprise a label.

The term nucleic acid sequence preferable refers to a DNA sequence or DNA molecule having the sequence as shown herein. This sequence can be used as a probe to detect HPV according to the invention.

In a preferred embodiment of the present invention, the probe is immobilised in a microarray. This microarray may also comprise other HPV probes known in the art. The supports that can be used and the microarray typology may vary, a selection being made amongst high and low density microarrays in a crystal support (CLART® Technology, shown for example in WO 2007/017699), or in a liquid support (Luminex Technology), but also amongst nitrocellulose macroarrays (Lipa Technology), and with a different labelling technology, such as labelling with fluorescence or precipitation of different compounds (Bodrossy & Sessitsch, 2004, Current Opinion in Microbiology, 7(3): 245-254).

In a preferred embodiment of the present invention the microarray has one or more nucleic acid probes immobilised on a plastic support, in particular, on a polystyrene support.

In a preferred embodiment of the present invention, the microarray comprises one or more nucleic acid probes designed for the detection of type 33 (Table 1), preferably the probe of SEQ ID N° 3. The microarray may further comprise one or more probes for the detection of one or more different HPV types, preferably up to a total of 35 different HPV types, as well as probes for the detection of one or more controls. Some examples of selectable probes are those disclosed in WO 2007/017699. The microarray may further comprise one or more position markers, which preferably are biotin-labelled probes.

In a preferred embodiment of the present invention, the microarray may be part of a slide or a reaction vessel. The latter may be in the form of an individual reaction tube, or in the form of a well within a set of reaction wells, which can be in the form of strips of wells or of plates. The latter may be constituted by independent wells, each of which comprising a microarray, and which may also be organised in the form of strips of wells.

Thereby, the microarrays of the present invention may be comprised in an individual reaction tube, in a strip of wells, each of the wells comprising a microarray, as well as in the form of a plate of wells. Preferably, the strip of wells contains 8 wells, and the plate of wells is in the form of a microtiter plate. In the most preferred embodiment, the microtiter plate is made of 96 wells. Each of the wells of the strip and of the plate, comprises a microarray.

In a preferred embodiment of the present invention, the probe microarray is immobilised on a solid support. This solid support may be contained in a reaction vessel, which can be an individual reaction tube, or be a well belonging to a strip of reaction wells, or to a plate of wells. According to another embodiment of the present invention, the solid support on which the microarray is immobilised, is the bottom of the reaction vessel itself. The reaction vessel may be in any of above-mentioned formats (an individual reaction tube, or a well within a set of reaction wells, which can be in the form of strips of wells or of plates).

In a preferred embodiment of the present invention, the DNA probes of the microarray are immobilised in the solid support by means of the presence of an amine group in the 5' end of the DNA probe.

In order to determine the HPV subtypes that are present within a sample, in a preferred embodiment of the aspects of the present invention, the sample is subjected to amplification prior to hybridisation with the type-specific probes. The amplification reaction is preferably PCR. Single stranded oligonucleotides may be obtained by denaturing any double stranded oligonucleotides present, for example by heating. Single stranded oligonucleotides are preferably allowed to hybridise under stringent conditions; such conditions will be understood to those of skill in the art, but preferably include incubating denatured oligonucleotides at 55°C with the target, in a buffer comprising 1 x SSC.Each amplification tube contains all the necessary reactives for carrying out a DNA amplification. In a preferred embodiment of the present invention, the reaction tube contains nucleotides (dNTPs), Taq polymerase enzyme, MgCl₂, enzyme buffer and a mixture of primers (preferably labelled with biotin), which are specific for the amplification of up to 35 HPV subtypes, according to literature (Manos et al., 1989, Cancer Cells, 7:209-214; Hildesheim et al., 1994, J. Infect. Dis., 169: 235-240). The strategy is based on the fact that the region to be amplified is highly conserved amongst the different HPV types to be detected, and on the use of degenerate primers that are suitable for amplifying above-mentioned subtypes.

In a preferred embodiment of the present invention, the system used for detection of the amplification products is based on the precipitation of an insoluble product in those spots of the microarray wherein hybridisation of the amplification products with their specific probes takes place. During PCR, the amplification products are labelled with biotin, either due to the fact that one or more of the primers are labelled with biotin in their 5' end, or due to the incorporation of biotin-labelled nucleotides. After amplification, the amplification products are hybridised with their complementary specific probes, which are immobilised in concrete and known spots of the microarray. Afterwards, incubation with a streptavidin-peroxidase conjugate takes place. The conjugate binds through its streptavidin moiety with the biotin of the amplification products (which are themselves bound to their specific probes), and the peroxidise activity results in the apparition of an insoluble product in the presence of the substrate TMB (3,3'5,5'-tetrametilbenzidina), which precipitates in the spots of the microarray wherein hybridisation takes place. Alternatively, o-Dianisidine may be used as substrate, as well as any other possible substrate which produces the same effect. The present invention admits any other possible way of labelling of the amplification products, as well as of visualization of their hybridisation with the corresponding DNA probes.

Preferably, the amplification tube of the present invention includes all the components necessary for carrying out a DNA extraction and amplification control.

In a preferred embodiment of the present invention, the amplification tube contains two primers, CFTR-F4 and CFTR-R5, which amplify a fragment of the human CFRT (Cystic Fibrosis Transmembrane Regulador) gene, of a length of 892 base pairs, which constitutes the genomic DNA extraction control. This control is necessary for the confirmation of a real negative result, as it informs of the presence of DNA of the patient in the sample, even though there has been no amplification of any HPV type. This same primers also amplify a region of 1202 base pairs of a recombinant plasmid, which has been constructed on the basis of the plasmid pBSK (pBluescript® II SK(+), Stratagene), and inserted in a pGEM-T(pGEM-T easy vector system, Promega), so as to constitute the amplification control of the tube. This construction is thus also contained within the PCR reaction mix.

This control will allow to distinguish between those cases of inhibition of the PCR reaction, and those in which there was no DNA present in the sample. The amplification reaction will always be unbalanced towards shorter DNA fragments, thereby favouring HPV amplification. Thus, according to the methods and kits of the invention, one or more control sequences may also be detected; for example, a probe immobilised to the solid support which does not hybridise to the target sequence from any HPV type. The probe may be for a human genomic target sequence; the assay may then comprise amplifying the human target sequence from the sample and detecting whether amplification has occurred. A further control may be introduced by using non-specific labelled sequences immobilised to the solid support; detection of the label can ensure that the label is working properly. A still further control may be provided by including a control amplification sequence which may be amplified by the same primers as the human target, but which will be detected by a different oligonucleotide on the solid support. This control ensures that amplification is working correctly.

The nucleic acid amplification mix used according to the methods and kits of the invention may comprise HPV consensus primers such as MY09 and MY11; and optionally HMB01; primers for amplifying a human target sequence; and a control amplification target sequence including sequences corresponding to flanking portions of the human target sequence, such that amplification of both target sequences will occur using the same primers. The kit may also include instructions for its use.

With the probes of Table 1, the non-specific hybridisation of the amplification product corresponding to type 31 HPV, and the probe corresponding to type 33 HPV, which took place when the microarray contained the original probes of SEQ ID NO 1 and 2, was avoided. However, with the probes of SEQ ID N° 4 to 7, the ability to bind type 33 HPV itself was lost. The reason for this is unknown, but it is not due to a lack of complementarity between the sequences of these probes and the sequence of the amplification product corresponding to type 33 HPV (Figure 2). The only probe that fulfils the purpose of eliminating the non-specific hybridisation between the amplification product corresponding to type 31 HPV and the probe corresponding to type 33 HPV, while conserving the ability to detect type 33 HPV itself, is the probe of SEQ ID N° 3 of Table 1.

One aspect of the present invention is a kit for the detection of one or more HPV types, the kit comprising a microarray which comprises or contains a nucleic acid probe comprising or consisting of the sequence CTGTCACTAGTTACTTGTGTGCA (5'→3') for the detection of type 33 HPV. This kit, as well as the microarray itself, constitute possible industrial applications of the present invention.

The kit according to the present invention may further comprise at least one probe selected from 31A-AS (TGTAGTATCACTGTTTGCAATTGCAGCACA (5'→3'), SEQ ID NO 8) and 31B5 (AGAACCTGAGGGAGGTGTGGTCAATCCAAA (5'→3'), SEQ ID N° 9) for detection of type 31 HPV. Other probes which may form part of the kit are described in the art, for example in WO 20071017699.

Further to the microarray, the kit of the present invention may comprise a mixture of reactives for the amplification of the DNA present in a sample and/or reactives for visualization of the hybridisation between the amplification product and the probes of the microarray.

### Examples.

The examples that are provided below merely illustrate the present invention. In no way do the technical aspects contained therein limit the scope of the invention.

### Example 1.

Studies carried out with real samples that were subjected to the detection method of WO 2007/017699, revealed that an unspecific hybridisation took place between the amplification product of genotype 31 HPV and the type-specific probe corresponding to genotype 33 HPV. Thus, when tested through nested-PCR and/or DNA sequencing, the samples that seemed to contain both genotypes 31 and 33 HPV, did in fact only contain type 31 HPV.

The sequences of probes known in the art (see WO 2007/017699) for detection of the PCR product of type 33 HPV are:

**Table 2**

| **SEQ ID N°** | **Probe Name** | **Sequence (5'→3')** | **nt N°** | **Mₜ*** | **%GC** |
|---|---|---|---|---|---|
| 1 | 33B1-AS | GTATATTTACCTAAGGGGTCTTCCTTTTCC | 30 | 84 | 40 |
| 2 | 33A2 | TACTGTCACTAGTTACTTGTGTGCATAAAG | 30 | 82 | 36,7 |

| | | | | | |
|---|---|---|---|---|---|
| *Mₜ: Melting Temperature | | | | | |

### Example 2

A total of 30 clinical samples were analysed, wherein the following kind of samples were represented:
(i) Samples which contained genotype 31, (ii) samples which contained genotype 33, (iii) samples wherein previous results provided a positive result for both genotype 31 and 33, but wherein the positive result corresponding to genotype 33 constituted a false positive of the technique, and, (iv) samples with a real coinfection of both the types 31 and 33.

With this purpose, DNA extraction of each sample was carried out, and the extracted material was subjected to PCR amplification. One part of the amplification product was hybridised to a microarray wherein the original probes of SEQ ID N° 1 and 2 (Table 2) were present for the detection of type 33 HPV; another part of the amplification product was hybridised to a microarray that contained the probe of SEQ ID N° 3 of the present invention (Table 1). Alternatively, microarrays that contained one or more of the probes of SEQ ID N° 4 to 7 (Table 1) were used; these microarrays provided a negative result in the case of samples which contained type 33 HPV (data not shown).

Finally, the presence of the insoluble product in those spots of the microarray wherein hybridisation between the amplification products and the probes of the microarray had taken place, was detected.

Each reaction tube contained all the reactives that were necessary for carrying out DNA amplification. In particular, nucleotides, (dNTPs), Taq polymerase enzyme, MgCl₂, enzyme buffer and a mixture of primers (labelled with biotin), which are specific for the amplification of up to 35 HPV subtypes, according to literature (Manos et al., 1989, Cancer Cells, 7:209-214; Hildesheim et al., 1994, J. Infect. Dis., 169: 235-240).

As a control, the amplification tube also contains two primers, CFTR-F4 and CFTR-R5, which amplify a fragment of the human CFRT (Cystic Fibrosis Transmembrane Regulador) gene, of a length of 892 base pairs, which constitutes the genomic DNA extraction control. This control is necessary for the confirmation of a real negative result, as it informs of the presence of DNA of the patient in the sample, even though there has been no amplification of any HPV type. This same primers also amplify a region of 1202 base pairs of a recombinant plasmid, which has been constructed on the basis of the plasmid pBSK (pBluescript® II SK(+), Stratagene), and inserted in a pGEM-T(pGEM-T easy vector system, Promega), so as to constitute the amplification control of the tube. This construction is thus also contained within the PCR reaction mix. This control will allow to distinguish between those cases of inhibition of the PCR reaction, and those in which there was no DNA present in the sample. The amplification reaction will always be unbalanced towards shorter DNA fragments, thereby favouring HPV amplification.

Visualisation of the hybrid constituted between the amplification product and the probe, was carried out through incubation with a streptavidin-peroxidase conjugate. The conjugate binds through its streptavidin moiety with the biotin of the amplification products (which are themselves bound to their specific probes). The peroxidise activity results in the apparition of an insoluble product in the presence of the substrate TMB (3,3'5,5'-tetrametilbenzidina), which precipitates in the spots of the microarray wherein hybridisation takes place.

Visualisation of two microarrays corresponding to a same sample was carried out in parallel.

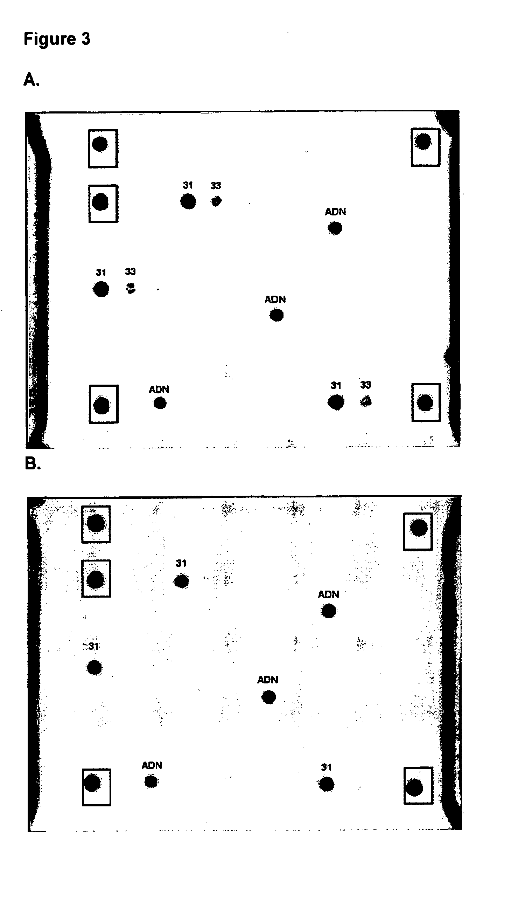

Figure 3 shows a representative example of the visualisation of the hybridisation of the amplification product corresponding to a sample, positive for type 31 HPV, and negative for type 33 HPV, and a microarray which contains original probes 33B1-AS and 33A2 for the detection of type 33 (panel A), and with a microarray containing the probe CTGTCACTAGTTACTTGTGTGCA (5'→3') according to the present invention, for detection of type 33 (panel B). The probes used for detection of type 31 HPV are probes 31A-AS and 31 B5. Both the microarrays of panel A and B comprise position markers, that are surrounded by squares, as well as probes for the detection of DNA amplification control, named as "ADN". Signals corresponding to the hybridisation signals of types 31 and 33 HPV are also indicated in this figure.

### Example 3.

In order to evaluate the functionality of the probe and microarray of the present invention within a detection system that further allows to identify other different HPV types, a set of 310 real samples, including samples which comprised a great number of different HPV genotypes, were analysed with a kit that comprised a microarray containing probe of SEQ ID N° 3 of the present invention for detection of type 33 HPV, probes of SEQ ID N° 8 and 9 for detection of type 31 HPV, and probes that were specific for the other HPV types to be detected.

Table 3 shows the result obtained for the different genotypes, as well as the specificity and sensitivity diagnostic parameters corresponding to each genotype.

**Table 3. Analysis of the diagnostic parameters of the Kit for detection of different HPV genotypes, based on the use of a microarray of probes that contains the probes of SEQ ID N° 3, 8 and 9 of the present invention. Column 1 indicates the genotype that is analysed. Columns 2 and 3 display the sensitivity and specificity values, respectively.**

| **Genotipe** | **Sensitivity** | **Specificity** |
|---|---|---|
| 6 | 94,12 | 100 |
| 11 | 100,00 | 100 |
| 16 | 100,00 | 100 |
| 18 | 100,00 | 100 |
| 26 | 100,00 | 100 |
| 31 | 100,00 | 100 |
| 33 | 100.00 | 100 |
| 35 | 100,00 | 100 |
| 39 | 100,00 | 100 |
| 40 | 100,00 | 100 |
| 42 | 100,00 | 100 |
| 43 | 100,00 | 100 |
| 44 | 100,00 | 100 |
| 45 | 100,00 | 100 |
| 51 | 100,00 | 100 |
| 52 | 100,00 | 100 |
| 53 | 96,97 | 100 |
| 54 | 100,00 | 100 |
| 56 | 100,00 | 100 |
| 58 | 96,30 | 100 |
| 59 | 100,00 | 100 |
| 61 | 100,00 | 100 |
| 62 | 94,12 | 100 |
| 66 | 100,00 | 100 |
| 68 | 100,00 | 100 |
| 70 | 100,00 | 100 |
| 71 | 100,00 | 100 |
| 72 | 100,00 | 100 |
| 73 | 100,00 | 100 |
| 81 | 100,00 | 100 |
| 82 | 100,00 | 100 |
| 83 | 100,00 | 100 |
| 84 | 94,44 | 100 |
| 89 | 100,00 | 100 |
| Total | 98.87 | 100 |

Application Project
   <120> Title :
   <130> AppFileReference :
   <140> currentAppNumber :
      <141> currentFilingDate :
Sequence
   <213> organismName : HPV
   <400> Presequencestring :
      gtatatttac ctaaggggtc ttccttttcc 30
   <212> Typo : DNA
      <211> Length : 30
      SequenceName : N° SEQ. 1 (33B1-AS)
      SequenceDescription : Probe
Sequence
   <213> organismName : HPV
   <400> PreSequenceString :
      tactgtcact agttacttgt gtgcataaag 30
   <212> Type : DNA
      <211> Length : 30
      SequenceName : N° SEQ. 2 (33A2)
      SequenceDescription : Probe
Sequence
   <213> organismName : HPV
   <400> PreSequenceString :
      ctgtcactag ttacttgtgt gca 23
   <212> Type : DNA
      <211> Length : 23
      SequenceName : N° SEQ. 3 (T33A2.1-A-AR)
      SequenceDescription : Probe
Sequence
   <213> OrganismName : HPV
   <400> PreSequenceString :
      gtatatttac ctaaggggtc 20
   <212> Type : DNA
      <211> Length : 20
      SequenceName : N° SEQ. 4 (T33B1.2-A-AR)
      SequenceDescription : Probe
Sequence
   <213> OrganismName : HPV
   <400> PreSequenceString :
      ccttttcctt tggaggtact g 21
   <212> Type : DNA
      <211> Length : 21
      SequenceName : N° SEQ. 5 (T33B1.3-A-AR)
      SequenceDescription : Probe
Sequence
   <213> OrganismName : HPV
   <400> PreSequenceString :
      gtatatttac ctaaggggtc ttcc 24
   <212> Type : DNA
      <211> Length : 24
      SequenceName : N° SEQ. 6 (T33B1.4-A-AR)
      SequenceDescription : Probe
Sequence
   <213> OrganismName : HPV
   <400> PreSequenceString :
      cttccttttc ctttggaggt actg 24
   <212> Type : DNA
      <211> Length : 24
      SequenceName : N° SEQ. 7 (T33B1.5-A-AR)
      SequenceDescription : Probe
Sequence
   <213> OrganismName : HPV
   <400> PreSequenceString :
      tgtagtatca ctgtttgcaa ttgcagcaca 30
   <212> Type : DNA

      <211> Length : 30
      SequenceName : No SEQ. 8 (31A-AS)
      SequenceDescription : Probe
Sequence
   <213> OrganismName : HPV
   <400> PreSequenceString :
      agaacctgag ggaggtgtgg tcaatccaaa 30
   <212> Type : DNA

      <211> Length : 30
      SequenceName : N° SEQ. 9 (31B5)
      SequenceDescription : Probe

## Claims

1. A nucleic acid consisting of sequence CTGTCACTAGTTACTTGTGTGCA (5'→3') (SEQ ID Nº 3).

2. Use of a nucleic acid sequence as defined in claim 1 for specific detection of type 33 HPV.

3. A microarray comprising one or more probes for the detection of one or more HPV types, **characterized in that** the microarray comprises a probe as defined in claim 1.

4. A microarray according to claim 3 further comprising at least one probe selected from the probes of sequence TGTAGTATCACTGTTTGCAATTGCAGCACA (5'→3') (SEQ ID Nº 8) and AGAACCTGAGGGAGGTGTGGTCAATCCAAA (5'→3') (SEQ ID Nº 9) for the detection of type 31 HPV.

5. A microarray according to claims 3 or 4 further comprising probes for the specific detection at least 5, 10, 15, 20, 25, 30, 35, 40, or 42 HPV types.

6. A kit for the detection of one or more HPV types in a sample, the kit comprising a microarray according to claims 3 to 5.

7. A kit according to claim 6 further comprising a mixture of reactives for the amplification of DNA present in the sample to be analysed, and/or reactives for the visualization of the hybridisation between the amplification product and the microarray probes.

8. A method for the specific detection of type 33 HPV comprising amplifying a DNA sample, obtaining single stranded oligonucleotides from said amplification product, and allowing said single stranded oligonucleotides to hybridise to a nucleic acid as defined in claim 1.

## Patentansprüche

1. Nucleinsäure, die aus der Sequenz CTGTCACTAGTTACTTGTGTGCA (5' →3') (SEQ ID Nr.: 3) besteht.

2. Verwendung einer in Anspruch 1 definierten Nucleinsäuresequenz zum spezifischen Nachweis von HPV Typ 33.

3. Mikroraster, das eine oder mehrere Sonden für den Nachweis eines oder mehrerer HPV-Typen aufweist, **dadurch gekennzeichnet, dass** das Mikroraster eine wie in Anspruch 1 definierte Sonde aufweist.

4. Mikroraster nach Anspruch 3, das ferner mindestens eine unter den Sonden der Sequenzen TGTAGTATCACTGTTTGCAATTGCAGCACA (5' →3') (SEQ ID Nr.: 8) und AGAACCTGAGGGAGGTGTGGTCAATCCAAA (5' →3') (SEQ ID Nr.: 9) ausgewählte Sonde für den Nachweis von HPV Typ 31 aufweist.

5. Mikroraster nach Anspruch 3 oder 4, das ferner Sonden für den spezifischen Nachweis von mindestens 5, 10, 15, 20, 25, 30, 35, 40 oder 42 HPV-Typen aufweist.

6. Kit für den Nachweis eines oder mehrerer HPV-Typen in einer Probe, wobei der Kit ein Mikroraster nach einem der Ansprüche 3 bis 5 aufweist.

7. Kit nach Anspruch 6, der ferner ein Gemisch von reaktiven Komponenten für die Amplifikation von DNA, die in der zu analysierenden Probe anwesend ist, und/oder reaktiven Komponenten für die Visualisierung der Hybridisierung zwischen dem Amplifikationsprodukt und den Mikroraster-Sonden aufweist.

8. Verfahren für den spezifischen Nachweis von HPV Typ 33, wobei das Verfahren aufweist: Amplifizieren einer DNA-Probe, Gewinnung einzelsträngiger Oligonucleotide aus dem Amplifikationsprodukt und Ermöglichen einer Hybridisierung der einzelsträngigen Oligonucleotide zu einer Nucleinsäure, wie in Anspruch 1 definiert.

## Revendications

1. Acide nucléique constitué de la séquence CTGTCACTAGTTACTTGTGTGCA (5'→3') (SEQ ID No. 3).

2. Utilisation d'une séquence d'acide nucléique telle que définie dans la revendication 1 pour une détection spécifique d'un HPV de type 33.

3. Microréseau comprenant une ou plusieurs sondes pour la détection d'un ou de plusieurs types de HPV, **caractérisé en ce que** le microréseau comprend une sonde telle que définie dans la revendication 1.

4. Microréseau selon la revendication 3, comprenant en outre au moins une sonde choisie parmi les sondes de séquence TGTAGTATCACTGTTTGCAATTGCAGCACA (5'→3') (SEQ ID No. 8) et AGAACCTGAGGGAGGTGTGGTCAATCCAAA (5'→3') (SEQ ID No. 9) pour la détection d'un HPV de type 31.

5. Microréseau selon la revendication 3 ou 4, comprenant en outre des sondes pour la détection spécifique d'au moins 5, 10, 15, 20, 25, 30, 35, 40 ou 42 types de HPV.

6. Kit pour la détection d'un ou de plusieurs types de HPV dans un échantillon, le kit comprenant un microréseau selon les revendications 3 à 5.

7. Kit selon la revendication 6, comprenant en outre un mélange de réactifs pour l'amplification d'un ADN présent dans l'échantillon à analyser et/ou de réactifs pour la visualisation de l'hybridation entre le produit d'amplification et les sondes du microréseau.

8. Procédé pour la détection spécifique d'un HPV de type 33 comprenant l'amplification d'un échantillon d'ADN, l'obtention d'oligonucléotides à brin simple à partir dudit produit d'amplification et l'étape consistant à permettre auxdits oligonucléotides à brin simple de s'hybrider à un acide nucléique tel que défini dans la revendication 1.
